# EUROPEAN PATENT APPLICATION

(11) **EP 2 984 962 A1**
(43) Date of publication of application: **17.02.2016**
(21) Application number: 15150916.3
(22) Date of filing: 13.01.2015
(51) Int. Cl.: A44C 15/00, A45D 33/33, A45D 34/02, A61L 9/12

(54) **Fragrant hanging ornament**

(30) Priority: 15.08.2014 TW 103214560
(71) Applicant: Glaspray Engineering & Manufacturing Co., Ltd., Tainan City, Chinese Taipei (CN)
(72) Inventor: Chen, Sin-Hsiung, Tainan City (TW)
(74) Representative: Hauck Patentanwaltspartnerschaft mbB

(57) **Abstract**

A fragrant hanging ornament has a shell (10), an absorbing element (20) and a ring (30). The shell (10) has a cover (12). The cover (12) has a positioning recess (121), a passage (122) and at least one outlet (123). The passage (122) is in communication with the positioning recess (121) and the at least one outlet (123). The absorbing element (20) is mounted in the positioning recess (121). A liquid fragrance product is filled into the absorbing element absorbs, and the scent of the liquid fragrance product is diffused through the passage (122) and out of the at least one outlet (123). A rope can be inserted through the ring (30), and the combination of the fragrant hanging ornament and the rope is taken as a necklace or a bracelet, so the fragrant hanging ornament is convenient to carry.

## Description

This application claims the benefit of the Taiwan patent application No. 103214560, filed on August 15, 2014, the entire contents of which are incorporated herein by reference.

### BACKGROUND OF THE INVENTION

### 1. Field of the Invention

The present invention relates to a fragrant hanging ornament, and more particularly to a fragrant hanging ornament that contains a liquid fragrance product and diffuses the scent of the liquid fragrance product.

### 2. Description of Related Art

Smell is one of human's perceptive senses, and each fragrance has a different effect, such as calming, relaxation and refreshment. A conventional fragrant diffuser is usually put in an indoor space. The fragrant diffuser has a container and a diffusing element. The container has a body, a chamber and an opening. The chamber is formed in the body and is filled with a liquid fragrance product. The opening is formed in a top surface of the body and is in communication with the chamber. The diffusing element is mounted on the container. A bottom end of the diffusing element is inserted through the opening of the container and into the chamber of the container, and is sunk in the liquid fragrance product. A top end of the diffusing element is revealed out of the opening of the container. The liquid fragrance product is siphoned from the bottom end of the diffusing element to the top end of the diffusing element. Therefore, the fragrant diffuser diffuses the scent of the liquid fragrance product by the diffusing element.

The volume of the fragrant diffuser is big. The weight of the container and the weight of the liquid fragrance product are heavy. The top end of the diffusing element is revealed out of the opening of the container. Therefore, the fragrant diffuser is inconvenient to carry, and the fragrant diffuser is only suitable for being placed in the indoor space. Thus, the scent of the liquid fragrance product diffused by the fragrant diffuser is kept in the indoor space, and users cannot smell the scent of the liquid fragrance product in the outdoors.

To overcome the shortcomings, the present invention tends to provide a fragrant hanging ornament to mitigate or obviate the aforementioned problems.

The main objective of the invention is to provide a fragrant hanging ornament that is convenient to carry.

The fragrant hanging ornament has a shell, an absorbing element and a ring. The shell has a seat, a chamber, a cover and a washer. The chamber is formed in the seat. The cover is mounted on the seat and has a positioning recess, a passage and at least one outlet. The positioning recess is formed on a bottom surface of the cover. The passage is formed in the cover and is in communication with the positioning recess. The at least one outlet is formed in an outside surface and each outlet is in communication with the passage. The washer is mounted on the cover and connected to the seat. The absorbing element is mounted in the shell and has a top end and a bottom end. The top end is mounted in the positioning recess of the cover. The bottom end is opposite to the top end and is inserted into the chamber of the seat. The ring is mounted on the cover of the shell.

A liquid fragrance product is filled into the absorbing element mounted in the cover. The cover is mounted on the seat, and the washer is connected to the cover and the seat The scent of the liquid fragrance product absorbed in the absorbing element is diffused through the passage of the cover and out of the at least one outlet of the cover. A rope can be inserted through the ring, and then the combination of the fragrant hanging ornament and the rope is regarded as a necklace or a bracelet, so the fragrant hanging ornament is convenient to carry. Furthermore, the scent of the liquid fragrance product is diffused out the shell continually, and users can smell the scent of the liquid fragrance product anywhere.

### IN THE DRAWINGS

- Fig. 1: is an exploded perspective view of a first embodiment of a fragrant hanging ornament in accordance with the present invention;
- Fig. 2: is a perspective view of the fragrant hanging ornament in Fig. 1;
- Fig. 3: is a side view in partial section of the fragrant hanging ornament in Fig. 2;
- Fig. 4: is an operational side view in partial section of the fragrant hanging ornament in Fig. 2, filled with the liquid fragrance product;
- Fig. 5: is an operational perspective view of the fragrant hanging ornament in Fig. 1, combined with a rope to serve as a necklace;
- Fig. 6: is another operational perspective view of the fragrant hanging ornament in Fig. 1, combined with a rope to serve as a bracelet;
- Fig. 7: is a perspective view of a second embodiment of a fragrant hanging ornament in accordance with the present invention; and
- Fig. 8: is a perspective view of a third embodiment of a fragrant hanging ornament in accordance with the present invention.

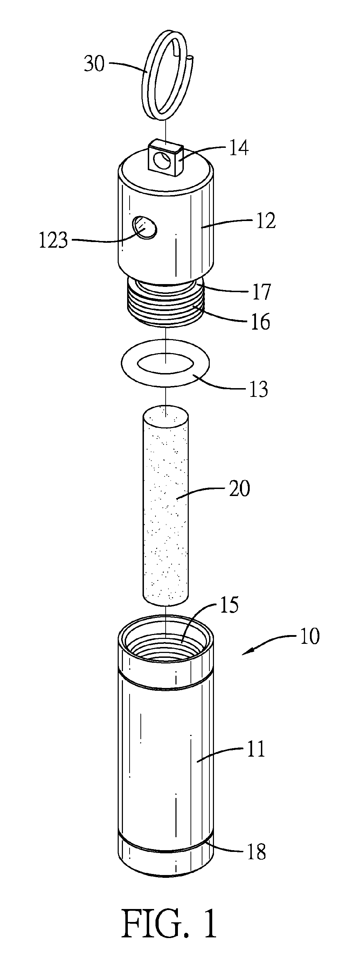

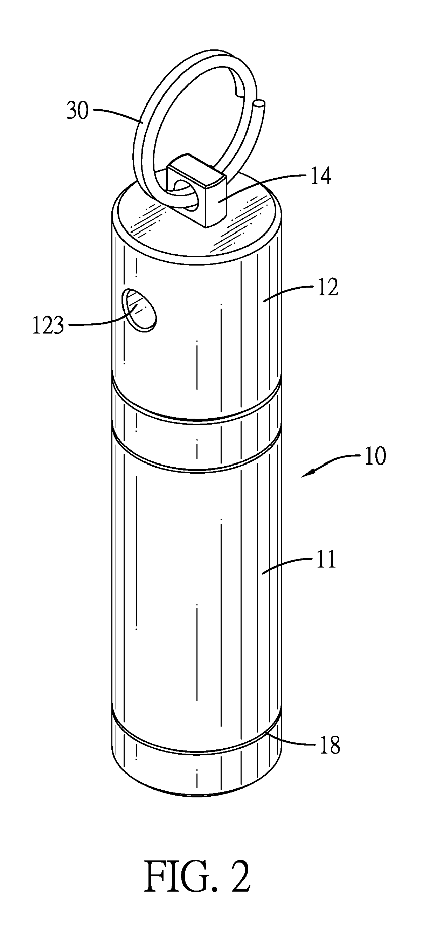

With reference to Figs. 1 to 3, a fragrant hanging ornament in accordance with the present invention comprises a shell 10, an absorbing element 20 and a ring 30.

The shell 10 has a seat 11, a chamber 111, a cover 12 and a washer 13. The chamber 111 is formed in the seat 11. The cover 12 is mounted on the seat 11. The cover 12 has a bottom surface, an outside surface, a positioning recess 121, a passage 122 and at least one outlet 123. The positioning recess 121 is formed on the bottom surface of the cover 12. The passage 122 is formed in the cover 12 and is in communication with the positioning recess 121. The at least one outlet 123 is formed in the outside surface of the cover 12 and each outlet 123 is in communication with the passage 122. The washer 13 is mounted on the cover 12 and is connected to the seat 11. Furthermore, the cover 12 has a lifting lug 14 formed on a top surface of the cover 12.

The absorbing element 20 is mounted in the shell 10. The absorbing element 20 has a top end and a bottom end. The top end is mounted in the positioning recess 121 of the cover 12. The bottom end is opposite to the top end and is inserted into the chamber 111 of the seat 11. The absorbing element 20 may be porous.

The ring 30 is mounted on the cover 12 of the shell 10. The ring 30 is circular and is mounted on the lifting lug 14 of the cover 12.

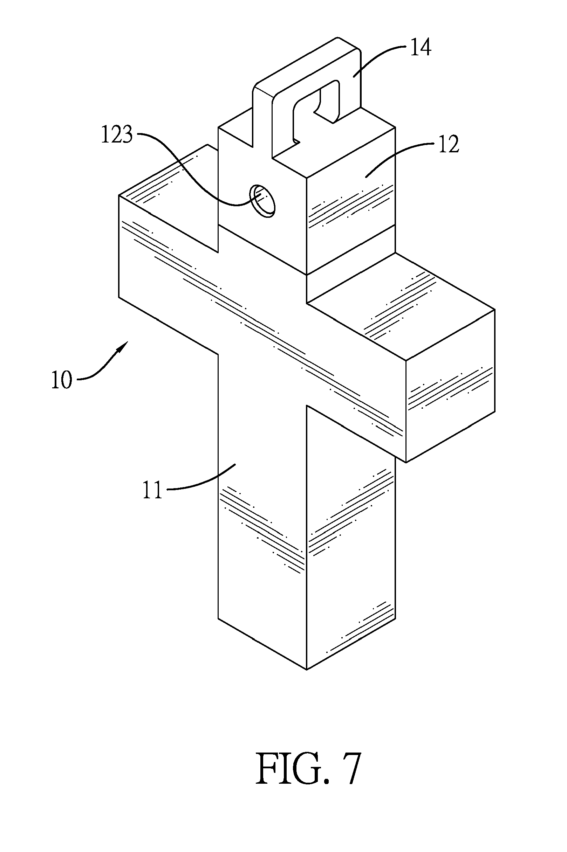

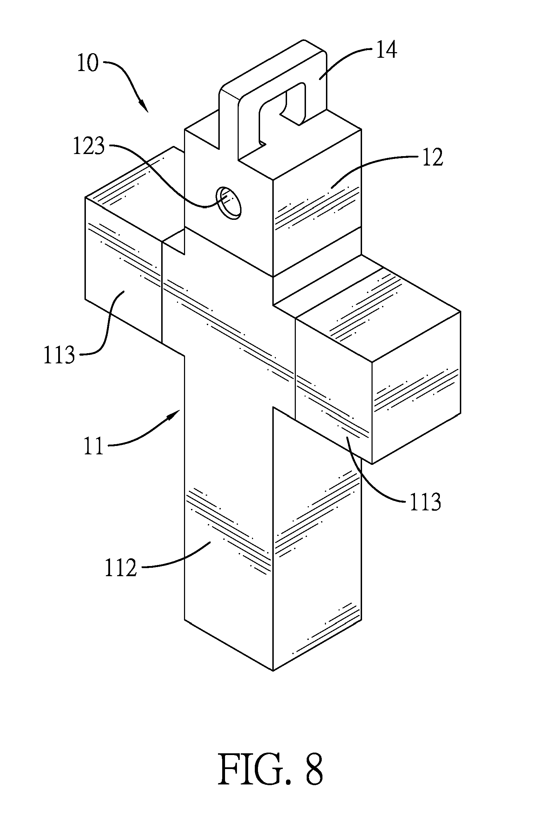

With reference to Fig. 1, in the first embodiment, the seat 11 is a cylindrical seat 11. With reference to Fig. 7, in the second embodiment, the seat 11 is T-shaped and is integrally formed as a single part. With reference to Fig. 8, in the third embodiment, the seat 11 is T-shaped and is composed of a pillar 112 and two arms 113. The pillar 112 has two outside surfaces opposite to each other. The arms 113 are respectively mounted on the outside surfaces of the pillar 112.

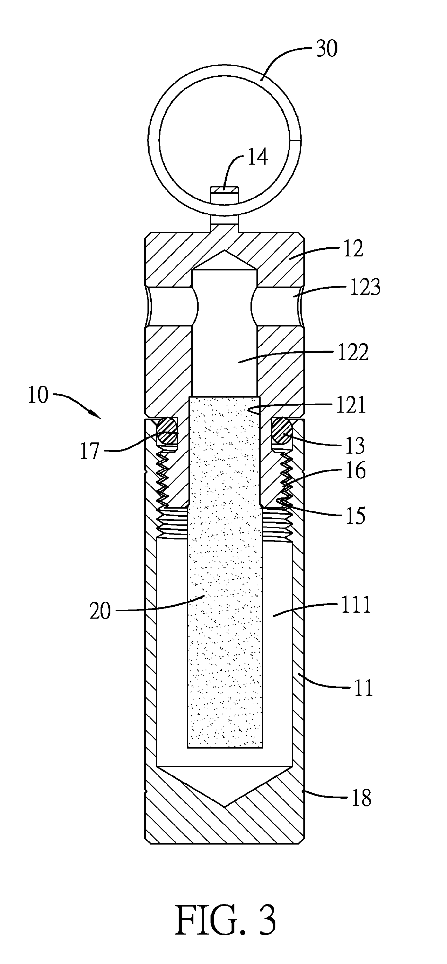

With reference to Figs. 1 and 3, the seat 11 has an internal thread 15 and the cover 12 has an external thread 16. The internal thread 15 of the seat 11 is formed on an inside surface of the seat 11. The external thread 16 of the cover 12 is formed on the outside surface of the cover 12 and is screwed with the internal thread 15 of the seat 11. The cover 12 has an annular groove 17 formed around the outside surface of the cover 12, and the washer 13 is mounted in the annular groove 17 of the cover 12. The seat 11 has a decorative line 18 formed on an outside surface of the seat 11.

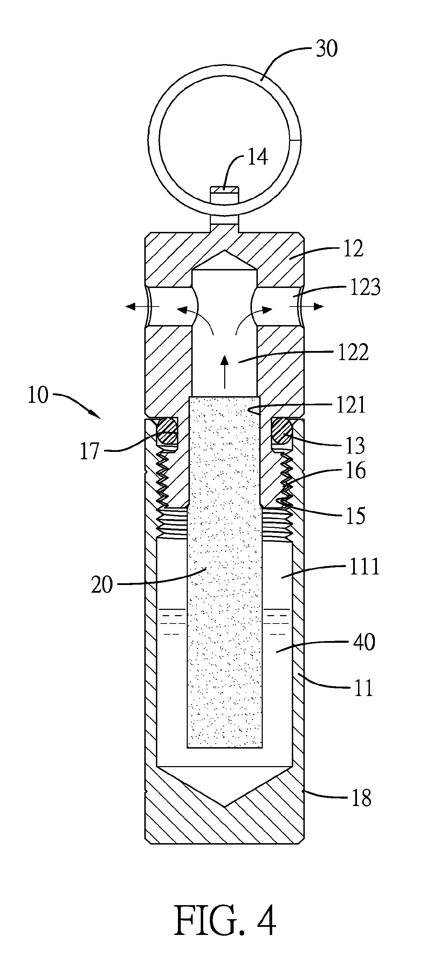

With reference to Fig. 4, a liquid fragrance product 40 is filled into the absorbing element mounted 20 in the cover 12. The cover 12 is mounted on the seat 11, and the washer 13 is connected to the cover 12 and the seat 11 The scent of the liquid fragrance product 40 absorbed by the absorbing element 20 is diffused through the passage 122 of the cover 12 and out of the at least one outlet 123 of the cover 12. Thus, the scent of the liquid fragrance product 40 is diffused out the fragrant hanging ornament under the circumstance that the cover 12 is mounted on the seat 11.

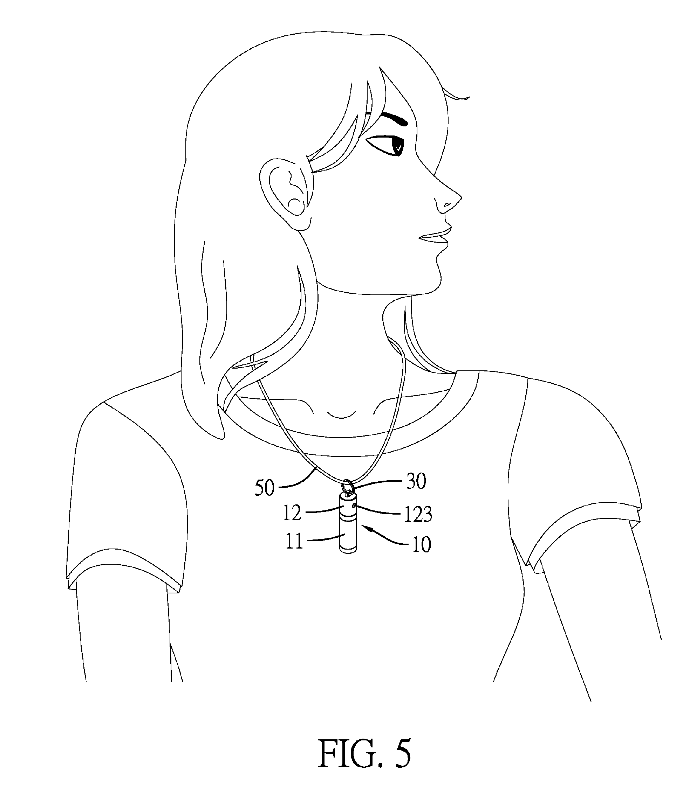

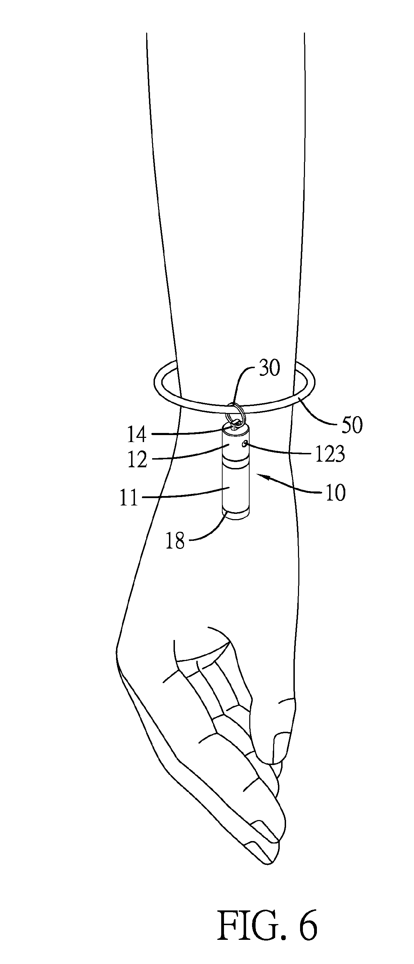

With reference to Figs. 5 and 6, a rope 50 can be inserted through the ring 30 of the fragrant hanging ornament, and then the combination of the fragrant hanging ornament and the rope 50 is taken as a necklace or a bracelet. Therefore, the fragrant hanging ornament is convenient to carry and the scent of the liquid fragrance product 40 is diffused out of the shell 10 continually. Users can smell the scent of the liquid fragrance product 40 at any desired place. Alternatively, users can open the cover 12 to smell the scent of the liquid fragrance product 40 absorbed in the absorbing element 20 directly. Users can change a new absorbing element 20 after the fragrant hanging ornament has been used for some time.

Accordingly, the fragrant hanging ornament is convenient to carry, and the scent of the liquid fragrance product 40 absorbed in the absorbing element 20 is diffused through the passage 122 of the cover 12 and out of the at least one outlet 123 of the cover 12. Users wearing the fragrant hanging ornament can smell the scent of the liquid fragrance product 40 at any desired time and place.

Even though numerous characteristics and advantages of the present invention have been set forth in the foregoing description, together with details of the structure and function of the invention, the disclosure is illustrative only, and changes may be made in detail, especially in matters of shape, size, and arrangement of parts within the principles of the invention to the full extent indicated by the broad general meaning of the terms in which the appended claims are expressed.

## Claims

1. A fragrant hanging ornament, **characterized in that** the fragrant hanging ornament comprises:
a shell (10) having a seat (11);
a chamber (111) formed in the seat (11);
a cover (12) mounted on the seat (11) and having a bottom surface;
an outside surface;
a positioning recess (121) formed on the bottom surface of the cover (12);
a passage (122) formed in the cover (12) and communicating with the positioning recess (121); and
at least one outlet (123) formed in the outside surface of the cover 12 and each outlet (123) communicating with the passage (122); and
a washer (13) mounted on the cover (12) and connected to the seat (11);
an absorbing element (20) mounted in the shell (10) and having a top end mounted in the positioning recess (121) of the cover (12); and
a bottom end opposite to the top end and inserted into the chamber (111) of the seat (11); and
a ring (30) mounted on the cover (12) of the shell (10).

2. The fragrant hanging ornament as claimed in claim 1, wherein the seat (11) is cylindrical.

3. The fragrant hanging ornament as claimed in claim 1, wherein the seat (11) is T-shaped.

4. The fragrant hanging ornament as claimed in claim 3, wherein the seat (11) is composed of a pillar (112) having two outside surfaces opposite to each other; and two arms (113) respectively mounted on the outside surfaces of the pillar (112).

5. The fragrant hanging ornament as claimed in any one of claims 1 to 4, wherein the seat (11) has an internal thread (15) formed on an inside surface of the seat (11); and the cover (12) has an external thread (16) formed on the outside surface of the cover (12) and screwed with the internal thread (15) of the seat (11).

6. The fragrant hanging ornament as claimed in claim 5, wherein the cover (12) has an annular groove (17) formed around the outside surface of the cover (12), and the washer (13) is mounted in the annular groove (17) of the cover (12).

7. The fragrant hanging ornament as claimed in any one of claims 1 to 6, wherein the cover (12) has a lifting lug (14) formed on a top surface of the cover (12), and the ring (30) is mounted on the lifting lug (14) of the cover (12).

8. The fragrant hanging ornament as claimed in claim 7, wherein the seat (11) has a decorative line (18) formed on an outside surface of the seat (11).
